# EUROPEAN PATENT APPLICATION

(11) **EP 4 282 417 A1**
(43) Date of publication of application: **29.11.2023**
(21) Application number: 23175318.7
(22) Date of filing: 25.05.2023
(51) Int. Cl.: A61K 31/4985, A61K 9/00, A61K 9/20, A61K 31/593, A61K 45/06, A61P 13/08

(54) **ASSOCIATION OF TADALAFIL AND VITAMIN D3 FOR USE IN THE ORAL TREATMENT OF BENIGN PROSTATIC HYPERTROPHY AND PRODUCTION METHODS THEREOF**

(30) Priority: 26.05.2022 IT 202200011114
(71) Applicant: Recordati Industria Chimica E Farmaceutica SPA, 20148 Milano (IT)
(72) Inventor: MONETTI, Vincenzo, I-06012 Citta' di Castello (PG) (IT)
(74) Representative: Viganò, Elena

(57) **Abstract**

An innovative formulation for the oral treatment of benign prostatic hypertrophy (BPH), which combines the unprecedented association of two acclaimed active ingredients such as Tadalafil and Vitamin D3, both effective in the prevention of erectile dysfunction, optionally combined with natural anti-inflammatory substances, such as Curcumin, Resveratrol and Lycopene, in order to exploit the synergistic effect of the two active ingredients in the treatment of benign prostatic hypertrophy, and also positively interfere in the chronic inflammation process that accompanies the same benign prostatic hypertrophy.

It is also an object of the invention the method for obtaining this formulation in different pharmaceutical forms of administration, such as tablets, capsules, or single-dose stick gel.

## Description

### Field of the invention

The present invention relates to an innovative formulation for the oral treatment of benign prostatic hypertrophy (BPH), which combines the unprecedented association of two acclaimed active ingredients such as Tadalafil and Vitamin D3, both effective in the prevention of erectile dysfunction, optionally combined with natural anti-inflammatory substances, such as Curcumin, Resveratrol and Lycopene, in order to exploit the synergistic effect of the two active ingredients in the treatment of benign prostatic hypertrophy, and also positively interfere in the chronic inflammation process that accompanies the same benign prostatic hypertrophy.

It is also an object of the invention the method for obtaining this formulation in different pharmaceutical forms of administration, such as tablets, capsules, or single-dose stick gel.

Benign prostatic hypertrophy (BPH), also simply called prostatic hypertrophy, is a benign enlargement of the prostate, with unique characteristics.

The prostate is a gland that is part of the male genital system, shaped like a chestnut, located below the bladder. It is crossed by the first section of the urethra, the tube that carries urine from the bladder to the outside. Benign prostatic hypertrophy consists of an enlargement of a portion of the gland (called transition zone) around the proximal part (closest to the bladder) of the prostatic urethra: this volumetric increase develops as a sleeve, increases the length of the urethra and, above all, prevents the urethra from widening into a funnel when the bladder should empty during urination.

Being a natural evolution of a portion of the prostate tissue in all men as they age, benign prostatic hypertrophy does not have a specific cause of its own: the factors (hormonal, biochemical, nutritional, etc.) that control prostate physiology, by evolving and changing as the age of the subject progresses, also govern its evolution in the development of hypertrophy.

Being a multifactorial process, for urologists the treatment of prostatic hypertrophy also includes the control of inflammation. In fact, benign prostatic hypertrophy arises from the persistence of a chronic state of alertness of the body, to which various factors may contribute: a bacterial or viral infection, hormonal or autoimmune alterations, the metabolic syndrome, or the physiological aging process (so much so that rates increase after the age of fifty).

The link of prostatic hypertrophy, at least in part, to a chronic inflammatory phenomenon is demonstrated by the fact that urologists use NSAIDs to decrease inflammation: in this regard, there is a product on the market to treat benign prostatic hypertrophy consisting of an extract of Serenoa Repens which inhibits the synthesis of interleukins, factors that induce inflammation.

See in this regard WO EP IT 258305B (Zambelletti SpA) which describes pharmaceutical compositions suitable for rectal administration, such as capsules of soft gelatin and suppositories containing Serenoa Repens extract as the active ingredient.

Currently, medical therapies are mainly based on the administration of:
**1) INHIBITORS of 5-A-REDUCTASE** (the enzyme that converts testosterone into DHT, the most active form of testosterone) which serve to slow down prostate growth, thus reducing the "fuel" of prostate cells: benign prostatic hypertrophy and prostate cells work with the same factors and use the same "fuel";
**2) TALADAFIL:** Tadalafil, like sildenafil and vardenafil, is a selective reversible inhibitor of phosphodiesterase type 5, present in high concentrations in the smooth muscle of the cavernous bodies of the penis where the enzyme promotes the degradation of cyclic guanosine monophosphate (cGMP). The increase in cGMP results in a relaxation of smooth muscle cells: vasodilation, which allows an increase in blood flow in the corpora cavernosa of the penis, is the basis of the erection mechanism. An increase in cGMP was also observed in the smooth muscles of the prostate, bladder, and the vasculature thereof.
   The vascular relaxation effect increases blood perfusion at the pelvic level and is associated with relaxation of the prostate and bladder smooth muscles; this mechanism of action is the basis of its use in the treatment of BPH symptoms (La Rivista/Anno 2014/Numero 2 del 2014: TADALAFIL NELL'IPERTROFIA PROSTATICA BENIGNA).
   This active ingredient has also proved effective against benign prostatic hyperplasia, so much so that it is sponsored by the European Medicines Agency (EMA) in the treatment of this disease. According to a document published in the European Urology journal and based on the uroflowmetric evaluations of some patients affected by the disease, tadalafil, like tamsulosin, also acts positively against the obstructive symptoms due to prostate enlargement, thus improving the maximum urinary flow rate.
3) **ALPHA-LYTICS:** Alpha-1 adrenergic receptor antagonists (alpha-lytics) are now considered as a first choice in the treatment of symptomatic benign prostatic hyperplasia (BPH). α1 adrenoceptors are involved in the regulation of many physiological processes: several studies have shown that these receptors are particularly represented in the prostatic stroma, in the bladder neck and in the prostatic urethra of many species, including humans. Alpha-lytics block the alpha receptors located on the prostate smooth muscle cells, obtaining the relaxation thereof, and favoring the funnel-shaped opening of the bladder neck and prostatic urethra. This improves thereof bladder emptying. These drugs are the first pharmacological approach to reduce urination frequency and improve flow.
**4) VITAMIN D3:** Vitamin D, known for its bone health benefits, also appears to play a role in the prevention of erectile dysfunction. Recent studies on the relationship between vitamin D and erectile dysfunction have shown that the deficiency of this vitamin is associated with an increased risk of ED. Additionally, some patients who do not benefit from PDE5i (phosphodiesterase 5 inhibitor) use, show low vitamin D levels (Kim HS, Cho MC. Low Serum 25-Hydroxyvitamin D Level as a Potential Risk Factor of Erectile Dysfunction in Elderly Men with Moderate to Severe Lower Urinary Tract Symptoms. World J Men's Health, 2022 Jan;40(1):139-148). This correlation is due to the protective effects of vitamin D on the cardiovascular system health which, consequently, has positive effects on the male sexual system health, but also to the involvement of vitamin D in the production of nitric oxide. As a proof of the importance of vitamin D, it is interesting to add that the testicle is an important site of activation of vitamin D, testifying to the fact that there is a close functional connection between vitamin D and the reproductive system (Dumbraveanu I, Banov P, Aria I, Ceban E. The Correlations of Clinical and Biochemical Indices of Vitamin D with Erectile Dysfunction. J Med Life. 2020 Apr-Jun;13(2):144-150).

From a chemical point of view, vitamin D plays an important role in the production of nitric oxide which is the main factor that favors the dilation of the arterial vessels of the heart, of the various vascular districts, and also of the penis, favoring an erection. Adequate levels of vitamin D appear to help reduce the risk of developing both cardiovascular disease and erectile dysfunction. Furthermore, vitamin D is able to reduce the presence in the blood of Sex Steroid Hormone-Binding Globulin (SHBG) which is a molecule capable of retaining free testosterone, the biologically active form of testosterone (Almeida Moreira Leal LK, et al. Vitamin D (VD3) antioxidative and anti-inflammatory activities: Peripheral and central effects. Eur J Pharmacol. 2020 Jul 15;879:173099).

Innovative formulations based on Tadalafil and Vitamin D have recently been proposed in the literature to exploit the synergistic and additional effects of the two compounds. See in this regard: Urol Int. 2021;105(5-6):514-519, "Whether Adding Vitamin D to Tadalafil 5 mg Treatment Is Useful in Patients with Erectile Dysfunction and Vitamin D Deficiency?", where the study carried out showed that daily oral administration of 5 mg of Tadalafil can have an additional positive effect on erectile function and sexual desire in patients.

In light of this knowledge, the Applicant has developed experimental research with the aim of developing a new composition for use in the treatment of benign prostatic hypertrophy, to be administered orally, with single daily doses, which includes the combination of Tadalafil and Vitamin D3, in order to exploit the enhanced effect deriving from the use of these two active ingredients in the specific treatment of benign prostatic hypertrophy.

Another aim of the research was to develop an orally administrable composition for use in the treatment of benign prostatic hypertrophy, which, in addition to the association between Tadalafil and Vitamin D3, also comprises a natural anti-inflammatory substance in combination, in order to simultaneously decrease/reduce also the inflammatory phenomenon that is associated with the same benign prostatic hypertrophy. In particular, this natural substance was selected among those having the greatest ability to positively interfere in the process, *i.e.* Curcumin, Resveratrol, and Lycopene.

Following a series of laboratory tests, the inventors verified that the two active ingredients, Tadalafil and Vitamin D3 can advantageously be produced in a tablet form using the traditional excipients reported in the current Pharmacopoeia, and that the same teachings can be used, in a simple and direct way, to obtain also the association of the two active principles with each of the substances of natural origin indicated above as tablets.

Four associations were thus identified:
Tadalafil + Vitamin D3
Tadalafil + Vitamin D3 + Resveratrol
Tadalafil + Vitamin D3 + Curcumin
Tadalafil + Vitamin D3 + Lycopene
of which, by way of example, some preferred formulations in weight per tablet are reported, where taken an ever-present amount of 5 mg/per tablet of Taladafil, the preferred amount of Vitamin D3 is of 0.05 mg/tablet, and can be in a range of 0.1 mg to 0.025 mg/tablet, corresponding to 4000-1000 international units.

**BASE ASSOCIATION: TADALAFIL + VITAMIN D3**

| **Ingredients** | **mg** | **Function** |
|---|---|---|
| Tadalafil | 5 | API |
| Vitamin D3 | 0.05 | API |
| Microcrystalline cellulose | 18 | Diluent |
| Sodium starch glycolate | 15 | Disintegrant |
| Magnesium stearate | 18 | Lubricant |
| Talc | 18 | Glidant |
| Total | 74.05 | |

**BASE ASSOCIATION: TADALAFIL + VITAMIN D3 + Resveratrol**

| **Ingredients** | **Mg** | **Function** |
|---|---|---|
| Tadalafil | 5 | API |
| Vitamin D3 | 0.05 | API |
| Resveratrol | 5 | Diluent |
| Microcrystalline cellulose | 18 | Diluent |
| Sodium starch glycolate | 15 | Disintegrant |
| Magnesium stearate | 18 | Lubricant |
| Talc | 18 | Glidant |
| Total | 79.05 | |

**BASE ASSOCIATION: TADALAFIL + VITAMIN D3 + Curcumin**

| **Ingredients** | **mg** | **Function** |
|---|---|---|
| Tadalafil | 5 | API |
| Vitamin D3 | 0.05 | API |
| Curcumin | 5 | Diluent |
| Microcrystalline cellulose | 18 | Diluent |
| Sodium starch glycolate | 15 | Disintegrant |
| Magnesium stearate | 18 | Lubricant |
| Talc | 18 | Glidant |
| Total | 79.05 | |

**BASE ASSOCIATION: TADALAFIL + VITAMIN D3 + Lycopene**

| **Ingredients** | **mg** | **Function** |
|---|---|---|
| Tadalafil | 5 | API |
| Vitamin D3 | 0.05 | API |
| Lycopene | 5 | Diluent |
| Microcrystalline cellulose | 18 | Diluent |
| Sodium starch glycolate | 15 | Disintegrant |
| Magnesium stearate | 18 | Lubricant |
| Talc | 18 | Glidant |
| Total | 79.05 | |

However, the Applicant has also found the need to improve the bioavailability of the same active ingredients, in consideration of the poor solubility and crystallinity of Tadalafil and Vitamin D3, characteristics resulting in poor wettability of the two active ingredients and poor absorption.

To overcome this obstacle, steps were initially taken to develop a procedure for making these two active ingredients wettable and amorphous in order to increase their release rates when they are administered in the form of tablets. This was achieved by making the tablet consist mainly of amorphous granules which are known to have a significantly higher absorption rate than a tablet without amorphous granules.

The production process developed according to the invention provides that:
the step of solubilization of the mixture of active ingredients, with the addition of Maltodextrin, according to a traditional procedure, in a hydroalcoholic solution, with a water/ethyl alcohol ratio of 10:90, is followed by a vacuum drying step using a rotavapor at 40/50°C, with the crucial presence of Vitamin E TPGS, which increases the wettability of the active ingredients, obtaining amorphous granules. The presence of the same Vitamin E TPGS gives the tablet thus obtained a dissolution rate higher than that of a tablet with maltodextrin alone.

Thus, the complete formulation of a tablet of the various associations is as follows:

### FORMULATION A:

**TABLET: TADALAFIL + VITAMIN D3 + Vitamin ETPGS IN AMORPHOUS GRANULES**

| **Ingredients** | **mg** | **Function** |
|---|---|---|
| Tadalafil | 5 | |
| Vitamin D3 | 0.05 | |
| Vitamin ETPGS | 1.00 | |
| Maltodextrins | 3.95 | |
| Total granules | 10 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 80 | |

### FORMULATION B relates to a:

**TABLET: TADALAFIL + VITAMIN D3 + Resveratrol + Vitamin ETPGS in AMORPHOUS GRANULES, and is as follows:**

| **Ingredients** | **mg** | **Function** |
|---|---|---|
| Tadalafil | 5 | |
| Vitamin D3 | 0.05 | |
| Vitamin ETPGS | 1.00 | |
| Resveratrol | 5.00 | |
| Maltodextrins | 8.95 | |
| Total granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

### FORMULATION C relates to a:

**TABLET: TADALAFIL + VITAMIN D3 + Curcumin + Vitamin ETPGS IN AMORPHOUS GRANULES, and is as follows:**

| **Ingredients** | **mg** | **Function** |
|---|---|---|
| Tadalafil | 5 | |
| Vitamin D3 | 0.05 | |
| Vitamin ETPGS | 1.00 | |
| Curcumin | 5.00 | |
| Maltodextrins | 8.95 | |
| Total granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

### FORMULATION D relates to a:

**TABLET: TADALAFIL + VITAMIN D3 + Lycopene + Vitamin ETPGS in AMORPHOUS GRANULES, and is as follows:**

| **Ingredients** | **mg** | **Function** |
|---|---|---|
| Tadalafil | 5 | |
| Vitamin D3 | 0.05 | |
| Vitamin ETPGS | 1.00 | |
| Lycopene | 5.00 | |
| Maltodextrins | 8.95 | |
| Total amorphous granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

It was then experimentally found that the procedure indicated above, in which vacuum drying with a rotavapor is essential to make the active ingredients amorphous, and the presence of Vitamin E TPGS to cover the maltodextrins with a film, so as to increase the dissolution rate and the absorption of the active ingredients, can be improved using the SPRAY DRYING technique which further micronizes the dispersed particles, and thus increases the amorphization and the dissolution capacity.

In fact, with this technique, by speeding up the drying process thanks to the pressure, it was possible to obtain:
amorphous granules, finer and more dispersible, and a further improvement in the tablets produced.

According to another aspect of the invention, the tablet formulation, described up to this point, was further enhanced from a pharmacological point of view by adding an amount of Magnesium Chloride, in consideration of the fact that Magnesium is recognized as having the ability to improve vasodilation of blood vessels.

The previous four formulations to which Magnesium Chloride was also added are reported below:

**TABLET: TADALAFIL + VITAMIN D3 + Vitamin ETPGS IN AMORPHOUS GRANULES + MgCl₂**

| **Ingredients** | **mg** | **Function** |
|---|---|---|
| Tadalafil | 5 | |
| Vitamin D3 | 0.05 | |
| Vitamin ETPGS | 1.00 | |
| Maltodextrins | 3.95 | |
| Total granules | 10 | |
| Magnesium chloride | 200 | API |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 280 | |

**TABLET: TADALAFIL + VITAMIN D3 + Resveratrol + Vitamin ETPGS IN AMORPHOUS GRANULES + MgCl₂**

| **Ingredients** | **mg** | **Function** |
|---|---|---|
| Tadalafil | 5 | |
| Vitamin D3 | 0.05 | |
| Vitamin ETPGS | 1.00 | |
| Resveratrol | 5.00 | |
| Maltodextrins | 8.95 | |
| Total granules | 20 | |
| Magnesium chloride | 200 | API |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 290 | |

**TABLET: TADALAFIL + VITAMIN D3 + Curcumin + Vitamin ETPGS IN AMORPHOUS GRANULES + MgCl₂**

| **Ingredients** | **mg** | **Function** |
|---|---|---|
| Tadalafil | 5 | |
| Vitamin D3 | 0.05 | |
| Vitamin ETPGS | 1.00 | |
| Curcumin | 5.00 | |
| Maltodextrins | 8.95 | |
| Total granules | 20 | |
| Magnesium chloride | 200 | API |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 290 | |

**TABLET: TADALAFIL + VITAMIN D3 + Lycopene + Vitamin ETPGS IN AMORPHOUS GRANULES + MgCl₂**

| **Ingredients** | **mg** | **Function** |
|---|---|---|
| Tadalafil | 5 | |
| Vitamin D3 | 0.05 | |
| Vitamin ETPGS | 1.00 | |
| Lycopene | 5.00 | |
| Maltodextrins | 8.95 | |
| Total granules | 20 | |
| Magnesium chloride | 200 | API |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 290 | |

### CAPSULES FORMULATION

Starting from this first positive result, derived from the amorphization procedure of the various active ingredients, Tadalafil and Vitamin D3 alone, or in association with natural substances having an anti-inflammatory effect, and also by adding Magnesium Chloride to affect vasodilation of blood vessels, the Applicant focused the interest on the production of other oral forms in order to broaden the commercial offer, improve the patient's adherence to therapy (compliance), improve the absorption of all the active ingredients, making them soluble in water, and mostly in order to be able to administer, with a single dose, greater amounts of natural substances with an anti-inflammatory effect, given that in the previously described tablet form the concentration of natural substances such as Resveratrol, Curcumin, Lycopene, cannot exceed the amount of 5 mg per tablet, as the amount of Vitamin E TPGS would be excessive and would not allow obtaining a compressible granule.

As an alternative to tablets, the Applicant has turned its attention to the development of a formulation for producing rigid capsules mainly comprised of the same amorphous granules, which maintains wettability and dispersion characteristics thereof while simplifying the production process.

In fact, the amorphous granules can be used as such to fill double-shell capsules, in suitable material (*e.g.* gelatin) further facilitating the administration of the active ingredients, Tadalafil and Vitamin D3 as such, alone or in association with a natural substance having anti-inflammatory effect selected from Resveratrol, Curcumin, and Lycopene.

Preferred formulations are given below by way of example:

**CAPSULE: TADALAFIL + VITAMIN D3 + Vitamin ETPGS IN AMORPHOUS GRANULES (gelatin)**

| **Ingredients** | **mg** | **Function** |
|---|---|---|
| Tadalafil | 5 | |
| Vitamin D3 | 0.05 | |
| Vitamin ETPGS | 1.00 | |
| Maltodextrins | 3.95 | |
| Total amorphous granules | 10 | |
| Lactose | 45 | Diluent |
| Corn starch | 45 | Diluent |
| Total | 100 | |

**CAPSULE: TADALAFIL + VITAMIN D3 + Resveratrol + Vitamin ETPGS IN AMORPHOUS GRANULES (gelatin)**

| **Ingredients** | **mg** | **Function** |
|---|---|---|
| Tadalafil | 5 | |
| Vitamin D3 | 0.05 | |
| Vitamin ETPGS | 1.00 | |
| Resveratrol | 5.00 | |
| Maltodextrins | 8.95 | |
| Total amorphous granules | 20 | |
| Lactose | 45 | Diluent |
| Corn starch | 45 | Diluent |
| Total | 110 | |

**CAPSULE: TADALAFIL + VITAMIN D3 + Curcumin + Vitamin ETPGS IN AMORPHOUS GRANULES (gelatin)**

| **Ingredients** | **mg** | **Function** |
|---|---|---|
| Tadalafil | 5 | |
| Vitamin D3 | 0.05 | |
| Vitamin ETPGS | 1.00 | |
| Curcumin | 5.00 | |
| Maltodextrins | 8.95 | |
| Total amorphous granules | 20 | |
| Lactose | 45 | Diluent |
| Corn starch | 45 | Diluent |
| Total | 110 | |

**CAPSULE: TADALAFIL + VITAMIN D3 + Lycopene + Vitamin ETPGS IN AMORPHOUS GRANULES (gelatin)**

| **Ingredients** | **mg** | **Function** |
|---|---|---|
| Tadalafil | 5 | |
| Vitamin D3 | 0.05 | |
| Vitamin ETPGS | 1.00 | |
| Lycopene | 5.00 | |
| Maltodextrins | 8.95 | |
| Total amorphous granules | 20 | |
| Lactose | 45 | Diluent |
| Corn starch | 45 | Diluent |
| Total | 110 | |

### FORMULATION IN GEL

Finally, we proceeded to increase the bioavailability of the active ingredients and natural molecules, namely Curcumin, Resveratrol and Lycopene, associated therewith, suggesting a composition obtained by solubilizing Tadalafil and Vitamin D3 together with one or more of the above natural molecules, in deionized water using a surfactant in the presence of a buffer, in order to form a single-dose stick gel where the concentration of Tadalafil is of 0.5 mg/ml and that of vitamin D3 is between 0.0025 and 0.005 mg/ml, equal to 0.025 mg and 0.05 mg in 10 of gel, respectively, corresponding to 1000 and 2000 IU.

This enables us to administer 5 mg of Taladafil with a gel single-dose of 10 ml.

In a preferred embodiment, vitamin E TPGS was used as surfactant together with excipients having a recognized mucoadhesive capacity.

Further characteristics and advantages of the invention will be highlighted by the following detailed description, in which the different operating steps and the experimental tests carried out are reported.

### DETAILED DESCRIPTION OF THE INVENTION

It was decided to start from a dosage equal to 5 mg of TADALAFIL and 0.05 mg of Vitamin D3, corresponding to 2,000 international units, to be administered by oral route.

### Experimental activities

A) A mixture of 5000 mg of Tadalafil, 50 mg of Vitamin D3, and 1000 mg of Vitamin ETPGS (agent that increases the wettability of the active ingredients) for a total of 6050 mg, was solubilized in a hydroalcoholic solution (water/ethyl alcohol, 10:90);
B) 3950 mg of Maltodextrins were added to the solution.
C) The suspension containing maltodextrins and the fully solubilized active ingredients and vitamin ETPGS was dried under vacuum by means of a rotavapor at 40-50°, obtaining amorphous granules.

Therefore, 10 mg of these amorphous granules contain 5 mg of Tadalafil, 0.05 mg of Vitamin D3, 1 mg of Vitamin E TPGS, and 3.95 mg of Maltodextrins.

Therefore, a complete formulation of a tablet of the different associations was obtained as follows:

### FORMULATION A) :

**TABLET: TADALAFIL + VITAMIN D3 + Vitamin ETPGS IN AMORPHOUS GRANULES**

| **Ingredients** | **mg** | **Function** |
|---|---|---|
| Tadalafil | 5 | |
| Vitamin D3 | 0.05 | |
| Vitamin ETPGS | 1.00 | |
| Maltodextrins | 3.95 | |
| Total granules | 10 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 80 | |

To highlight the effect of the amorphous granules, the dissolution rate was determined following the traditional procedure described by the Pharmacopoeia of Tablets containing Maltodextrins only (the formulation is reported below) and those containing the amorphous granules.

**TABLET: TADALAFIL + VITAMIN D3 + MALTODEXTRINS**

| **Ingredients** | **mg** | **Function** |
|---|---|---|
| Tadalafil | 5 | |
| Vitamin D3 | 0.05 | |
| Maltodextrins | 4.95 | |
| Total | 10 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 80 | |

Taladafil release was measured over time, as determined by HPLC.

| Time | % release Tablet with MALTODEXTRINS | % release Tablet with AMORPHOUS GRANULES |
|---|---|---|
| 0 | 0 | 0 |
| 5 | 4 | 10 |
| 10 | 9 | 20 |
| 15 | 19 | 42 |
| 20 | 35 | 75 |
| 25 | 40 | 90 |
| 30 | 45 | 95 |

The above table demonstrates that the presence of the amorphous granules and the presence of Vitamin E, TPGS, a surfactant that improves wettability, speeds up the release of TADALAFIL.

This is due to the fact that the active ingredients were made amorphous, by drying under vacuum in the crucial presence of Vitamin ETPGS.

This result should be considered very positive.

For this reason, the same procedure was applied to the other associations:
B) Tadalafil + Vitamin D3 + Resveratrol + amorphous granules
C) Tadalafil + Vitamin D3 + Curcumin + amorphous granules, and
D) Tadalafil + Vitamin D3 + Lycopene + amorphous granules
as will be described in more detail in the following.

In particular:

### B) Resveratrol Granules:

The procedure used is briefly described below:

A mixture of 5000 mg of Tadalafil, 50 mg of Vitamin D, 5000 mg of Resveratrol, and 1000 mg of Vitamin ETPGS (wettability enhancing agent), (for a total of 11050 mg) was solubilized in a hydroalcoholic solution - water/ethyl alcohol, 10:90; and
8950 mg of Maltodextrins were added to the solution.

The suspension containing maltodextrins and the fully solubilized active ingredients and vitamin ETPGS was dried under vacuum by means of a rotavapor at 40-50°C obtaining amorphous granules.

Therefore, 20 mg of these amorphous granules contain 5 mg of Tadalafil, 0.05 mg of Vitamin D3, 5 mg of Resveratrol, 1 mg of Vitamin E TPGS, and 8.95 mg of Maltodextrins.

### FORMULATION B relating to a:

**TABLET: TADALAFIL + VITAMIN D3 + Resveratrol + Vitamin ETPGS IN AMORPHOUS GRANULES, is as follows:**

| **Ingredients** | **mg** | **Function** |
|---|---|---|
| Tadalafil | 5 | |
| Vitamin D3 | 0.05 | |
| Vitamin ETPGS | 1.00 | |
| Resveratrol | 5.00 | |
| Maltodextrins | 8.95 | |
| Total granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

### FORMULATION C:

### Curcumin granules:

The procedure used is briefly described below:
a) A mixture of 5000 mg of Tadalafil, 50 mg of Vitamin D3, 5000 mg of Curcumin, and 1000 mg of Vitamin ETPGS (agent that increases the wettability of the active ingredients) (a total of 11050 mg), was solubilized in a hydroalcoholic solution (water/ethyl alcohol, 10:90);
b) 8950 mg of Maltodextrins were added to said solution.

The suspension containing Maltodextrins and the fully solubilized active ingredients and Vitamin ETPGS was dried under vacuum by means of a rotavapor at 40-50°C, obtaining AMORPHOUS GRANULES.

20 mg of these AMORPHOUS GRANULES contain 5 mg of Tadalafil, 0.05 mg of Vitamin D3, 5 mg of Curcumin, 1 mg of Vitamin E TPGS and 8.950 mg of Maltodextrins.

### FORMULATION C relating to a:

**TABLET: TADALAFIL + VITAMIN D3 + Curcumin + Vitamin ETPGS IN AMORPHOUS GRANULES is as follows:**

| **Ingredients** | **mg** | **Function** |
|---|---|---|
| Tadalafil | 5 | |
| Vitamin D3 | 0.05 | |
| Vitamin ETPGS | 1.00 | |
| Curcumin | 5.00 | |
| Maltodextrins | 8.95 | |
| Total granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

### FORMULATION D:

### Lycopene Granules:

The procedure used is briefly described below:

A mixture of 5000 mg of Tadalafil, 50 mg of Vitamin D3, 5000 mg of Lycopene, and 1000 mg of Vitamin ETPGS (agent that increases the wettability of the active ingredients) (a total of 11050 mg) was solubilized in a hydroalcoholic solution (water/ethyl alcohol, 10:90);
8950 mg of Maltodextrins were added to said solution.

The suspension containing maltodextrins and the fully solubilized active ingredients and vitamin ETPGS was dried under vacuum by means of a rotavapor at 40-50°C, obtaining amorphous granules.

20 mg of these amorphous granules contain 5 mg of Tadalafil, 0.05 mg of Vitamin D3, 5 mg of Lycopene, 1 mg of Vitamin E TPGS and 8.950 mg of Maltodextrins.

Therefore, the **FORMULATION D relating to a:**

**TABLET: TADALAFIL + VITAMIN D3 + Lycopene + Vitamin ETPGS IN AMORPHOUS GRANULES, is as follows:**

| **Ingredients** | **mg** | **Function** |
|---|---|---|
| Tadalafil | 5 | |
| Vitamin D3 | 0.05 | |
| Vitamin ETPGS | 1.00 | |
| Lycopene | 5.00 | |
| Maltodextrins | 8.95 | |
| Total granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

The formulations containing the AMORPHOUS GRANULES indicated above were subjected to the dissolution test as previously indicated.

Also in this case, the release rate of TADALAFIL was faster than traditional formulations containing maltodextrins only.

For clarity, TRADITIONAL formulations CONTAINING Maltodextrins only are reported below:

**TABLET: TADALAFIL + VITAMIN D3 + Resveratrol**

| **Ingredients** | **mg** | **Function** |
|---|---|---|
| Tadalafil | 5 | |
| Vitamin D3 | 0.05 | |
| Resveratrol | 5.00 | |
| Maltodextrins | 9.95 | |
| Total granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

**TABLET: TADALAFIL + VITAMIN D3 + Curcumin**

| **Ingredients** | **mg** | **Function** |
|---|---|---|
| Tadalafil | 5 | |
| Vitamin D3 | 0.05 | |
| Curcumin | 5.00 | |
| Maltodextrins | 9.95 | |
| Total granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

**TABLET: TADALAFIL + VITAMIN D3 + Lycopene**

| **Ingredients** | **mg** | **Function** |
|---|---|---|
| Tadalafil | 5 | |
| Vitamin D3 | 0.05 | |
| Lycopene | 5.00 | |
| Maltodextrins | 9.95 | |
| Total granules | 20 | |
| Microcrystalline cellulose | 19 | Diluent |
| Sodium starch glycolate | 15 | Disintegrant |
| Magnesium stearate | 18 | Lubricant |
| Talc | 18 | Glidant |
| Total | 90 | |

Once the procedure for obtaining tablets and capsules had been defined, the Applicant set out to verify the possibility of obtaining a product in the form of a mucoadhesive gel which could be packaged in single-dose sticks, possibly associating Tadalafil and Vitamin D3 with a natural substance such as curcumin, resveratrol, or lycopene.

The characteristics of the two active ingredients, TADALAFIL and VITAMIN D3, are reported below. 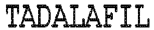 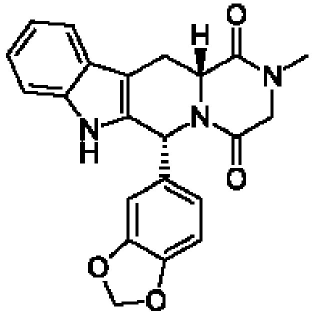
6*R*-(benzo[*d*][1,3]dioxol-5-yl)-2-methyl-2,3,6,7,1212a*R-*hexahydropirazino[1',2':1,6]pirido[3,4*b*]indole-1,4-dione
Brute or Molecular Formula: C₂₂H₁₉N₃O₄
Molecular weight (u): 389.41
CAS No. 171596-29-5

Tadalafil is insoluble in water, poorly soluble in ethanol and propylene glycol, soluble in polyethylene glycol-400 and in Transcutol. 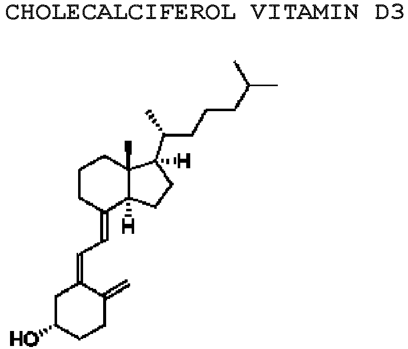
Brute or Molecular Formula: C₂₇H₄₄O
Molecular Weight (u): 384.64 g/mol
CAS No. 67-97-0
Vitamin D3 is practically insoluble in water.

### Characteristics of ancillary substances

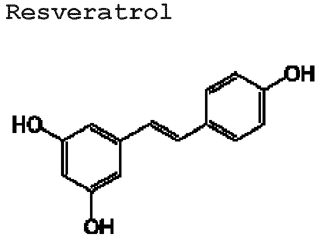

- Resveratrol is insoluble in water.
- The anti-inflammatory properties of Resveratrol make this substance useful even in case of chronic pain due to inflammatory processes. 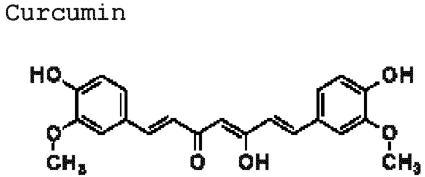 Enol form
- Curcumin is insoluble in water.
- Anti-inflammatory activity: it reduces the expression of enzymes involved in the development of the inflammatory reaction. 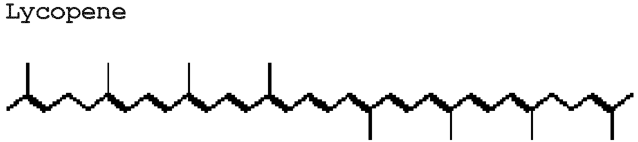
- Lycopene is insoluble in water.
- It is the carotenoid molecule most present in the human body.

### EXPERIMENTAL ACTIVITY

The operational steps through which the formulations according to the invention were defined will now be described.

### Preliminary Activity

A dosage equal to 5 mg of TADALAFIL and 0.05 mg of Vitamin D3, equal to 2000 international units, to be administered orally was identified.

Various tests of Tadalafil solubilization in water at different concentrations, under different pH conditions, were carried out using phosphate buffers, tris HCl, etc., with the aim of having even partial solubilization:

### First Test

| **Ingredients** | **Concentration (%)** | |
|---|---|---|
| Tadalafil | 0.500 | 5 mg/ml |
| Citrate Buffer q.s. to pH | 5.5-6 | |
| Deionized water q.s. to | 100 ml | |
| Presence of precipitate | yes | |

### Second Test

| **Ingredients** | **Concentration (%)** | |
|---|---|---|
| Tadalafil | 0.05 | 0.5 mg/ml |
| Citrate Buffer *q.s*. to pH | 5.-6 | |
| Deionized water *q.s*. to | 100 ml | |
| Presence of precipitate | yes | |

### Third Test

| **Ingredients** | **Concentration (%)** | |
|---|---|---|
| Tadalafil | 0.005 | 0.05 mg/ml |
| Citrate Buffer *q.s.* to pH | 5.5-6 | |
| Deionized water *q.s.* to | 100 ml | |
| Presence of precipitate | yes | |

The same tests were carried out with phosphate buffer at pH 7-7.5 obtaining the same negative result.

It was then decided to try the solubilization of Tadalafil using surfactants, such as Solutol SH and Vitamin E TPGS

### Test no. 1

| **Ingredients** | **Concentration (%)** | |
|---|---|---|
| Tadalafil | 0.500 | 5 mg/ml |
| Solutol SH | 1.0 | |
| Citrate Buffer *q.s*. to pH | 5.5-6 | |
| Deionized water *q.s*. to | 100 ml | |
| Presence of precipitate | yes | |

### Test no. 2

| **Ingredients** | **Concentration (%)** | |
|---|---|---|
| Tadalafil | 0.500 | 5 mg/ml |
| Solutol SH | 2.0 | |
| Citrate Buffer *q.s*. to pH | 5.5-6 | |
| Deionized water *q.s.* to | 100 ml | |
| Presence of precipitate | si | |

### Test no. 3

| **Ingredients** | **Concentration (%)** | |
|---|---|---|
| Tadalafil | 0.50 | 5 mg/ml |
| Solutol SH | 3.0 | |
| Citrate Buffer *q.s*. to pH | 5.5-6 | |
| Deionized water *q.s*. to | 100 ml | |
| Presence of precipitate | Yes | |

### Test no. 4

| **Ingredients** | **Concentration (%)** | |
|---|---|---|
| Tadalafil | 0.050 | 0.5 mg/ml (10 ml =5 mg) |
| Solutol SH | 4.0 | |
| Citrate Buffer *q.s.* to pH | 5.5-6 | |
| Deionized water *q.s.* to | 100 ml | |
| Opalescent solution | Opalescent | |

### Test no. 5

| **Ingredients** | **Concentration (%)** | |
|---|---|---|
| Tadalafil | 0.050 | 0.5 mg/ml (10 ml=5 mg) |
| Solutol SH | 5.0 | |
| Citrate Buffer *q.s.* to pH | 5.5-6 | |
| Deionized water *q.s.* to | 100 ml | |
| Solution | Slightly Opalescent | |

Test no. 5 gives a fairly good positive indication, since with the administration of 10 ml of opalescent solution, 5 mg of Tadalafil are dosed, which is the amount prescribed for the oral treatment of BPH by the European Medicines Agency (EMA).

Test no. 5 is important because it is positive from the solubilization point of view, but it was not considered completely acceptable because Solutol SH is particularly bitter, and therefore difficult to use for oral administration especially if it has to be at a concentration of the order of 5%. Therefore, another surfactant was selected, Vitamin E TPGS, which is not bitter, and has an excellent ability to form micelles which allow the solubilization of insoluble substances. Furthermore, Vitamin ETPGS has the advantage that its solubilization capacity is also combined with a recognized ability to improve intestinal absorption of the solubilized substances, which in the specific case described, has a significant importance.

### Test no. 1

| **Ingredients** | **Concentration (%)** | |
|---|---|---|
| Tadalafil | 0.500 | 5 mg/ml |
| Vitamin E TPGS | 1.0 | |
| Citrate Buffer *q.s.* to pH | 5.5-6 | |
| Deionized water *q.s.* to | 100 ml | |
| Presence of precipitate | yes | |

### Test no. 2

| **Ingredients** | **Concentration (%)** | |
|---|---|---|
| Tadalafil | 0.500 | 5 mg/ml |
| Vitamin E TPGS | 2.0 | |
| Citrate Buffer *q.s.* to pH | 5.5-6 | |
| Deionized water *q.s*. to | 100 ml | |
| Presence of precipitate | yes | |

### Test no. 3

| **Ingredients** | **Concentration (%)** | |
|---|---|---|
| Tadalafil | 0.050 | 0.5 mg/ml (10 ml=5 mg) |
| Vitamin E TPGS | 3.0 | |
| Citrate Buffer *q.s.* to pH | 5.5-6 | |
| Deionized water *q.s.* to | 100 ml | |
| Solution | Opalescent | |

### Test no. 4

| **Ingredients** | **Concentration (%)** | |
|---|---|---|
| Tadalafil | 0.050 | 0.5 mg/ml (10 ml= 5 mg) |
| Vitamin E TPGS | 4.0 | |
| Citrate Buffer *q.s*. to pH | 5.5-6 | |
| Deionized water *q.s.* to | 100 ml | |
| Solution | Slightly opalescent | |

With **Test no. 5,** the **BASE FORMULATION** was achieved:

| **Ingredients** | **Concentration (%)** | |
|---|---|---|
| Tadalafil | 0.050 | 0.5 mg/ml (10 ml =5 mg) |
| Vitamin E TPGS | 5.0 | |
| Citrate Buffer *q.s.* to pH | 5.5-6 | |
| Deionized water *q.s.* to | 100 ml | |
| Solution | Clear | |

- The use of 5% Vitamin E TPGS has, in fact, provided an excellent solubilization, superior to the solubilization previously obtained with Solutol HS15.
- According to the invention, Vitamin D3 was added to the BASE FORMULATION as the second active ingredient in the primary association of TADALAFIL and VITAMIN D3.

### FORMULATION: TADALAFIL + VITAMIN D3 ASSOCIATION

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Citrate Buffer *q.s*. to pH | 5.5-6 | |
| Deionized water *q.s*. to | 100 ml | |
| Solution | Clear | |

However, the tests carried out highlighted that the concentration of Vitamin D3 can also be lower than 0.005 mg/ml, equal to 0.05 mg in 10 ml, for example 0.0025 mg/ml equal to 0.025 mg in 10 ml, or even 0.0015 mg/ml equal to 0.015 mg in 10 ml, while keeping the solution always clear.

The result is positive: there is complete solubilization of Tadalafil and Vitamin D3.

At this point, having defined the base formulation, an anti-inflammatory component was added to the association "Tadalafil and Vitamin D3", introducing substances of natural origin, such as Curcumin, Resveratrol and Lycopene in the formulation indicated above.

### Formulations with Curcumin

### Formulation: ASSOCIATION OF TADALAFIL, Vitamin D3 and 0.250% Curcumin

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.250 | 2.5 mg/ml (10 ml=25 mg) |
| Citrate Buffer *q.s.* to pH | 5.5-6 | |
| Deionized water *q.s.* to | 100 ml | |
| Solution | Clear | Deep yellow color |

### Formulation: ASSOCIATION OF TADALAFIL, Vitamin D3 and 0.350% Curcumin

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.350 | 3.5 mg/ml (10 ml=35 mg) |
| Citrate Buffer *q.s.* to pH | 5.5-6 | |
| Deionized water *q.s.* to | 100 ml | |
| Solution | Clear | Deep yellow color |

### Formulation: ASSOCIATION OF TADALAFIL, Vitamin D3 and 0.400% Curcumin

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.40 | 4.0mg/ml (10 ml=35 mg) |
| Citrate Buffer *q.s*. to pH | 5.5-6 | |
| Deionized water *q.s.* to | 100 ml | |
| Solution | Clear | Deep yellow color |

### Formulation: ASSOCIATION OF TADALAFIL, Vitamin D3 and 0.500% Curcumin

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.500 | 5.0 mg/ml (10 ml=50mg) |
| Citrate Buffer *q.s.* to pH | 5.5-6 | |
| Deionized water *q.s.* to | 100 ml | |
| Solution | Clear | Deep yellow color |

### Formulations with Resveratrol

### Formulation: ASSOCIATION OF TADALAFIL, Vitamin D3 and 0.250% Resveratrol

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.250 | 2.5 mg/ml (10 ml=25 mg) |
| Citrate Buffer *q.s.* to pH | 5.5-6 | |
| Deionized water *q.s.* to | 100 ml | |
| Solution | Clear | |

### Formulation: ASSOCIATION OF TADALAFIL, Vitamin D3 and 0.350% Curcumin

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.3500 | 3.5 mg/ml (10 ml=35 mg) |
| Citrate Buffer *q.s.* to pH | 5.5-6 | |
| Deionized water *q.s.* to | 100 ml | |
| Solution | Clear | |

### Formulation: ASSOCIATION OF TADALAFIL, Vitamin D3 and 0.5% Resveratrol

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.5 | 5.0 mg/ml (10 ml=50mg) |
| Citrate Buffer *q.s*. to pH | 5.5-6 | |
| Deionized water *q.s.* to | 100 ml | |
| Solution | Clear | |

As can be seen from the data shown, the use of Vitamin E TPGS has therefore provided positive results, allowing to reach an oral formulation in which a fundamental sweetish taste characteristic is added to a good solubilization, combined with a recognized ability to improve the absorption of water insoluble principles, as confirmed by patients.

### PREPARATION OF GEL FORMULATIONS

To further improve what was previously obtained, a certain amounts of Poloxamer 407, an excipient which has a recognized mucoadhesive capacity combined with a good solubilizing capacity, was added to the formulations in order to obtain a stable mucoadhesive gel.

### Formulation: ASSOCIATION OF TADALAFIL, Vitamin D3 with 2.5% Poloxamer 407

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Poloxamer 407 | 2.5 | |
| Citrate Buffer *q.s.* to pH | 5.5-6 | |
| Deionized water *q.s.* to | 100 ml | |
| Solution | Light clear gel | |

### Formulation: ASSOCIATION OF TADALAFIL, Vitamin D3, 0.400% Curcumin and 2.5% Poloxamer

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.40 | 4.0mg/ml (10 ml= 40 mg) |
| Poloxamer 407 | 2.5 | |
| Citrate Buffer *q.s.* to pH | 5.5-6 | |
| Deionized water *q.s.* to | 100 ml | |
| Appearance | Light clear gel | Deep yellow color |

### Formulation: ASSOCIATION OF TADALAFIL, Vitamin D3, 0.500% Curcumin and 2.5% Poloxamer

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.500 | 5.0 mg/ml (10 ml= 50 mg) |
| Poloxamer 407 | 2.5 | |
| Citrate Buffer *q.s.* to pH | 5.5-6 | |
| Deionized water *q.s.* to | 100 ml | |
| Appearance | Light clear gel | Deep yellow color |

### Formulation: ASSOCIATION OF TADALAFIL, Vitamin D3, 0.350% Resveratrol with 2.5% Poloxamer

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.3500 | 3.5 mg/ml (10 ml= 35 mg) |
| Poloxamer 407 | 2.5 | |
| Citrate Buffer *q.s.* to pH | 5.5-6 | |
| Deionized water *q.s.* to | 100 ml | |
| Appearance | Light clear gel | Colorless |

### Formulation: ASSOCIATION OF TADALAFIL, Vitamin D3, 0.4% Resveratrol with 2.5% Poloxamer

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.4 | 4.0mg/ml (10 ml= 40 mg) |
| Poloxamer 407 | 2.5 | |
| Citrate Buffer *q.s.* to pH | 5.5-6 | |
| Deionized water *q.s.* to | 100 ml | |
| Appearance | Light clear gel | Colorless |

### Formulation: ASSOCIATION OF TADALAFIL, Vitamin D3, 0.5% Resveratrol with 2.5% Poloxamer

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.5 | 5 mg/ml (10 ml= 50 mg) |
| Poloxamer 407 | 2.5 | |
| Citrate Buffer *q.s.* to pH | 5.5-6 | |
| Deionized water *q.s*. to | 100 ml | |
| Appearance | Light clear gel | Colorless |

The formulations were checked for 15 days at room temperature. After this period, the formulations remained clear, but the consistency of the gel was very low, almost zero. It was therefore decided to suitably modify the gelling system, replacing Poloxamer 407 with Xanthan Gum, a polysaccharide with a good ability to viscose and form stable and mucoadhesive gels.

The formulations are reported below.

### JELLIFICATION WITH XANTHAN GUM

### Formulation: ASSOCIATION OF TADALAFIL, Vitamin D3 with 1.5% Xanthan Gum

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin ETPGS | 5.0 | |
| Xanthan Gum | 1.5 | |
| Citrate Buffer *q.s.* to pH | 5.5-6 | |
| Deionized water *q.s.* to | 100 ml | |
| Appearance | Compact gel | |

### Formulation: ASSOCIATION OF TADALAFIL, Vitamin D3 with 2.5% Xanthan Gum

| Ingredients | Concentration (%) | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin ETPGS | 5.0 | |
| Xanthan Gum | 2.5 | |
| Citrate Buffer *q.s.* to pH | 5.5-6 | |
| Deionized water *q.s.* to | 100 ml | |
| Appearance | Very compact gel | |

### Formulation: ASSOCIATION OF TADALAFIL, Vitamin D3, 0.500% Curcumin with 1.5% Xanthan Gum

| **Ingredients** | **Concentration (%)** | |
|---|---|---|
| Tadalafil | 0.050 | .5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.500 | 5.0 mg/ml (10 ml= 50 mg) |
| Xanthan Gum | 1.5 | |
| Citrate Buffer *q.s.* to pH | 5.5-6 | |
| Deionized water *q.s.* to | 100 ml | |
| Appearance | Compact uniform gel | Deep Yellow Color |

### Formulation: ASSOCIATION OF TADALAFIL, Vitamin D3, 0.500% Curcumin with 2.5% Xanthan Gum

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.500 | 5.0 mg/ml (10 ml= 50 mg) |
| Xanthan Gum | 2.5 | |
| Citrate Buffer *q.s*. to pH | 5.5-6 | |
| Deionized water *q.s*. to | 100 ml | |
| Appearance | Very compact uniform gel | Deep Yellow Color |

### Formulation: ASSOCIATION OF TADALAFIL, Vitamin D3, 0.500% Resveratrol with 1.5% Xanthan Gum

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.5 | 5 mg/ml (10 ml= 50 mg) |
| Xanthan Gum | 1.5 | |
| Citrate Buffer *q.s.* to pH | 5.5-6 | |
| Deionized water *q.s.* to | 100 ml | |
| Appearance | Compact gel | |

### Formulation: ASSOCIATION OF TADALAFIL, Vitamin D3, 0.500% Resveratrol with 2.5% Xanthan Gum

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.5 | 5 mg/ml (10 ml= 50 mg) |
| Xanthan Gum | 2.5 | |
| Citrate Buffer *q.s.* to pH | 5.5-6 | |
| Deionized water *q.s.* to | 100 ml | |
| Appearance | Very compact gel | |

With the addition of Xanthan Gum, gels became very compact and uniform both at the concentration of 1.5% and 2.5%.

It can therefore be concluded that the formulations containing 0.05% of Tadalafil, 0.005% of Vitamin D3 *per se* or also in the presence of Curcumin, Resveratrol, and Lycopene, respectively, once viscosized with Xanthan Gum are transformed into a compact and uniform gel, which can be packaged in single-dose sticks, having a pleasant taste, to be ingested orally, possibly using a sweetener such as sucralose in the presence of curcumin or resveratrol.

The mucoadhesive gel formulation was also further optimized from a PHARMACOLOGICAL point of view because an amount of MAGNESIUM CHLORIDE comprised between 200-800 mg was added to the formulations indicated.

Below are the complete formulations with magnesium chloride at a concentration of 4%.

### Formulation: ASSOCIATION OF TADALAFIL, Vitamin D3 with 1.5% Xanthan Gum + MgCl₂

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Magnesium chloride | 2-8 | 200-800 mg in 10 ml |
| Xanthan Gum | 1.5 | |
| Citrate Buffer *q.s.* to pH | 5.5-6 | |
| Deionized water *q.s*. to | 100 ml | |
| Appearance | Compact gel | |

### Formulation: ASSOCIATION OF TADALAFIL, Vitamin D3 with 2.5% Xanthan Gum + MgCl₂

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Magnesium chloride | 2-8 | 200-800 mg in 10 ml |
| Xanthan Gum | 2.5 | |
| Citrate Buffer *q.s.* to pH | 5.5-6 | |
| Deionized water *q.s*. to | 100 ml | |
| Appearance | Very compact gel | |

### Formulation: ASSOCIATION OF TADALAFIL, Vitamin D3, 0.500% Curcumin with 1.5% Xanthan Gum + MgCl₂

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.500 | 5.0 mg/ml (10 ml= 50 mg) |
| Magnesium chloride | 2-8 | 200-800 mg in 10 ml |
| Xanthan Gum | 1.5 | |
| Citrate Buffer *q.s.* to pH | 5.5-6 | |
| Deionized water *q.s*. to | 100 ml | |
| Appearance | Compact uniform gel | Deep Yellow Color |

### Formulation: ASSOCIATION OF TADALAFIL, Vitamin D3, 0.500% Curcumin with 2.5% Xanthan Gum + MgCl₂

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Curcumin | 0.500 | 5.0 mg/ml (10 ml= 50 mg) |
| Magnesium chloride | 2-8 | 200-800 mg in 10 ml |
| Xanthan Gum | 2.5 | |
| Citrate Buffer *q.s.* to pH | 5.5-6 | |
| Deionized water *q.s*. to | 100 ml | |
| Appearance | Very compact uniform gel | Deep Yellow Color |

### Formulation: ASSOCIATION OF TADALAFIL, Vitamin D3, 0.500% Resveratrol with 1.5% Xanthan Gum + MgCl₂

| **Ingredients** | **Concentration (%)** | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.5 | 5 mg/ml (10 ml= 50 mg) |
| Magnesium chloride | 2-8 | 200-800 mg in 10 ml |
| Xanthan Gum | 1.5 | |
| Citrate Buffer *q.s.* to pH | 5.5-6 | |
| Deionized water *q.s.* to | 100 ml | |
| | Compact gel | |

### Formulation: ASSOCIATION OF TADALAFIL, Vitamin D3, 0.500% Resveratrol with 2.5% Xanthan Gum

| Ingredients | Concentration (%) | |
|---|---|---|
| Tadalafil | 0.050 | 0.5 mg/ml (10 ml =5 mg) |
| Vitamin D3 | 0.0005 | 0.005 mg/ml (10 ml = 0.05 mg) |
| Vitamin E TPGS | 5.0 | |
| Resveratrol | 0.5 | 5 mg/ml (10 ml= 50 mg) |
| Magnesium chloride | 2-8 | 200-800 mg in 10 ml |
| Xanthan Gum | 2.5 | |
| Citrate Buffer *q.s*. to pH | 5.5-6 | |
| Deionized water *q.s.* to | 100 ml | |
| Solution | Very compact gel | |

It should be noted that magnesium chloride can be replaced by other magnesium salts, such as: magnesium citrate, magnesium lactate, magnesium pyruvate, magnesium pidolate, magnesium gluconate, etc.

## Claims

1. A composition containing the association of the two active ingredients Tadalafil and Vitamin D3 for use in the treatment of benign prostatic hypertrophy.

2. The composition for use according to claim 1, wherein said active ingredients, Tadalafil and Vitamin D3, are associated with a molecule of natural origin, selected from Resveratrol, Curcumin and Lycopene, having the effect of decreasing the chronic inflammation associated with benign prostatic hypertrophy.

3. The composition for use according to claim 2, **characterized in that** the associations thus obtained are produced in the form of tablets, each of which contains:
- 1 to 5 mg of Tadalafil, and 0.1 to 0.05 mg of Vitamin D3, or
- 1 to 5 mg of Tadalafil, and 0.1 to 0.05 mg of Vitamin D3 + 5 mg of Resveratrol; or
- 1 to 5 mg of Tadalafil, and 0.1 to 0,05 mg of Vitamin D3 + 5 mg of Curcumin; or
- 1 to 5 mg of Tadalafil, and 0.1 to 0.05 mg of Vitamin D3 + 5 mg of Lycopene.

4. The composition for use according to claim 1, **characterized in that** it is formulated in the form of a tablet containing amorphous granules, consisting of 5 mg of Tadalafil, 0.05 mg of Vitamin D3, 1 mg of Vitamin E TPGS and 3.95 mg of Maltodextrins, for a total of 10 mg, and by other excipients that form the tablet structure with a final weight of 80 mg, wherein the dissolution rate is higher compared to the formulation without amorphous granules.

5. The composition for use according to claim 2, **characterized in that** it is formulated in the form of a tablet containing amorphous granules, consisting of 5 mg of Tadalafil, 0.05 mg of Vitamin D3, 5 mg of Resveratrol, 1 mg of Vitamin E TPGS, and 8.950 of Maltodextrins for a total of 20 mg, and by other excipients that form the tablet structure, wherein the dissolution rate is higher compared to the formulation without amorphous granules.

6. The composition for use according to claim 5, **characterized in that** it is formulated in the form of a tablet containing amorphous granules consisting of 5 mg of Tadalafil, 0.05 mg of Vitamin D3, 5 mg of Resveratrol, 1 mg of Vitamin E TPGS, and 8.950 of Maltodextrins for a total of 20 mg, and by other excipients that form the tablet structure, wherein Resveratrol is replaced by 5 mg of Curcumin.

7. The composition for use according to claim 5, **characterized in that** it is formulated in the form of a tablet containing amorphous granules, consisting of 5 mg of Tadalafil, 0.05 mg of Vitamin D3, 5 mg of Resveratrol, 1 mg of Vitamin E TPGS, and 8.950 of maltodextrin for a total of 20 mg, and by other excipients that form the tablet structure, wherein Resveratrol is replaced by 5 mg of Lycopene.

8. Method of preparing amorphous granules containing the association of the two active ingredients Tadalafil and Vitamin D3, to be used for the preparation of tablets for the treatment of benign prostatic hypertrophy, **characterized in that** it involves the steps of:
- solubilizing said active ingredients, optionally together with Resveratrol or Curcumin or Lycopene, in a hydroalcoholic solution wherein the water to ethyl alcohol ratio is equal to 10/90;
- adding Maltodextrins, and
- eliminating ethyl alcohol under vacuum.

9. Gel composition comprising Tadalafil and Vitamin D3, **characterized in that** said active ingredients, Tadalafil and Vitamin D3, are solubilized in deionized water by means of a surfactant in the presence of a buffer to increase their bioavailability.

10. The gel composition according to claim 9, **characterized in that** the concentration of Tadalafil is of 0.5 mg/ml, equal to 5 mg in 10 ml.

11. The gel composition according to claim 9, **characterized in that** the concentration of vitamin D3 is comprised between 0.005 mg/ml, equal to a dose of 0.05 mg in 10 ml, equal to 2000 IU, and 0.0025 mg/ml, equal to a dose of 0.025 mg in 10 ml, equal to 1000 IU.

12. The gel composition according to claim 9, **characterized in that** the surfactant is Vitamin E TPGS, preferably in a concentration equal to 5% by weight, or SOLUTOL SH15.

13. The gel composition according to claim 9, **characterized in that** the deionized aqueous solution is buffered at pH 5.5-6.

14. The gel composition according to claim 9, **characterized in that** it further contains at least one of the natural substances capable of decreasing the state of inflammation associated with prostatic hypertrophy, wherein said natural substances, having the effect of decreasing the state of inflammation, are selected from: Resveratrol, Curcumin, and Lycopene, and are also solubilized in the same buffered aqueous solution using the same surfactant.

15. The gel composition according to claim 14, **characterized in that** it comprises Curcumin or Resveratrol or Lycopene in a concentration comprised between 0.250 and 0.5%, *i.e.* from 2.5 mg/ml to 5 mg/ml.

16. The gel composition according to claim 9, **characterized in that** it comprises Xanthan Gum in a concentration comprised between 1.5 and 2.5% by weight.

17. Single-dose stick gel for use in the oral treatment of benign prostatic hypertrophy, BPH, containing:
| **Ingredients** | **Concentration (% w/w)** | |
|---|---|---|
| Tadalafil | 0.050 | 0.5 mg/ml (10ml =5mg) |
| Vitamin D3 | 0.0005 | 0.005 mg/ml (10ml =0.05mg) |
| Vitamin E TPGS | 5.0 | |
| Xanthan Gum | 1.5 - 2.5 | viscosifier |
| Citrate buffer *q.s.* to pH | 5.5-6 | |
| Deionized water *q.s.* to | 100 ml | |

18. Single-dose stick gel for use in the oral treatment of benign prostatic hypertrophy, BPH, containing:
| **Ingredients** | **Concentration (% w/w)** | |
|---|---|---|
| Tadalafil | 0.050 | 0.5 mg/ml (10 ml =5 mg) |
| Vitamin D3 | 0.0005 | 0.005 mg/ml (10 ml =0.05mg) |
| Vitamin E TPGS | 5.0 | |
| Curcumin or Resveratrol or Lycopene | 0.500 | 5.0 mg/ml (10 ml= 50 mg) |
| Xanthan Gum | 1.5 - 2.5 | Viscosifier |
| Citrate buffer *q.s.* to pH | 5.5-6 | |
| Sucralose | 0.02-0.04 | Sweetener* |
| Preservative System | | |
| Sodium benzoate | 0.2 | |
| Sodium sorbate | 0.2 | |

19. Method of preparing a single-dose stick gel for the oral treatment of benign prostatic hypertrophy according to claim 17 or 18, **characterized in that** it comprises the following steps:
A) Tadalafil, at a concentration of 0.050%, and Vitamin D3, at a concentration of 0.0005%, are solubilized in deionized water using an amount of Vitamin E equal to 5% of the total weight, in the presence of buffer *q.s.* to pH 5.5-6;
B) optionally, an anti-inflammatory component consisting of at least one natural anti-inflammatory substance selected from Curcumin, Resveratrol and Lycopene, at a concentration ranging from 0.250 to 0.500%, is added to said base solution;
C) the formulation thus obtained is gelified with xanthan gum at a concentration comprised between 1.5% and 2.5%, transforming it into a compact and uniform gel.
